(19) Europäisches Patentamt / European Patent Office / Office européen des brevets

(11) **EP 4 215 176 A1**

(12) **EUROPEAN PATENT APPLICATION**
published in accordance with Art. 153(4) EPC

(43) Date of publication:
26.07.2023 Bulletin 2023/30

(21) Application number: 21869265.5

(22) Date of filing: 08.09.2021

(51) International Patent Classification (IPC):
*A61K 8/29* (2006.01)          *A61K 8/02* (2006.01)
*A61Q 1/12* (2006.01)          *C01B 25/37* (2006.01)

(52) Cooperative Patent Classification (CPC):
**A61K 8/0254; A61K 8/02; A61K 8/29; A61Q 1/02;
C01B 25/37;** A61K 2800/412; A61Q 1/12

(86) International application number:
**PCT/JP2021/033013**

(87) International publication number:
**WO 2022/059578 (24.03.2022 Gazette 2022/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 18.09.2020 JP 2020157503

(71) Applicant: **Fujimi Incorporated**
**Kiyosu-shi, Aichi 452-8502 (JP)**

(72) Inventors:
• **IWAKUNI Mayumi**
**Kiyosu-shi, Aichi 452-8502 (JP)**
• **ASHITAKA Keiji**
**Kiyosu-shi, Aichi 452-8502 (JP)**
• **MIWA Naoya**
**Kiyosu-shi, Aichi 452-8502 (JP)**

(74) Representative: **Maiwald GmbH**
**Elisenhof**
**Elisenstraße 3**
**80335 München (DE)**

(54) **POWDER, WHITE PIGMENT FOR COSMETIC, AND COSMETIC**

(57)    Provided is a powder having excellent hiding power, such that the powder can be used in place of titanium dioxide as a white pigment in a cosmetic. The powder of the present invention includes plate-shaped crystal particles, the volume D50% diameter thereof is 0.7 um or more and 8.0 um or less, and a coefficient of variation (CV value) of a primary particle diameter is 1.0 or less.

EP 4 215 176 A1

EP 4 215 176 A1

**Description**

Technical Field

The present invention relates to powder, white pigment for a cosmetic, and a cosmetic.

Background Art

The white pigment for a cosmetic is a base pigment when obtaining a cosmetic composition by mixing with other pigments. Conventionally, as a white pigment for a cosmetic, titanium oxide (IV) (TiO$_2$, titanium dioxide; hereinafter, also simply referred to as "titanium oxide") is generally used. The reason is that titanium dioxide has a high refractive index and is excellent in hiding power.

PTL 1 discloses rutile-type titanium oxide aggregated particles characterized in that the aggregated particles are formed by further aggregating fan-shaped rutile-type titanium oxide particles in which rod-shaped particles having a side size of 0.05 um to 0.2 um and a dimension in thickness direction of 0.02 um to 0.1 um are gathered and/or bonded to each other, and the aggregated particles have a particle diameter of 0.1 um to 5.0 um, and an average friction coefficient (MIU value) of 0.2 or more and less than 0.7. According to the rutile-type titanium oxide aggregated particles, it is described that when the aggregated particles are blended in a cosmetic, particularly a make-up cosmetic, the cosmetic can be smoothly applied to the skin, and there is no squeaky or rough feeling on the skin, and an effect of producing a natural feeling of bare skin can be exerted without whitening, by appropriate coloring power and hiding power.

PTL 2 describes an oil-based solid cosmetic that contains (A) 5 to 40% by weight of one or two or more solid waxes selected from a microcrystalline wax, a polyethylene-polypropylene copolymer, a polyethylene wax, a ceresin wax, and a paraffin wax, (B) 1% to 30% by weight of one or two or more fine particle powders selected from aluminum oxide and silicic acid anhydride, which has a particle diameter of 0.1 um or less, and (C) 30% to 80% by weight of a liquid oil agent, in which the oil-based solid cosmetic has a glossiness of 40% or more in a case where both an incident angle and a reflection angle are 60°.

PTL 3 describes a solid make-up and/or care cosmetic composition in the form of a compact powder, which contains, in a physiologically acceptable medium, at least one pulverulent phase, one emulsifier, one hydrophilic gelling agent, one organic lake, at least one organopolysiloxane elastomer, and one hydrophilic active agent with hygroscopic property, the hydrophilic active agent being present in a content between 1% by mass and 40% by mass with respect to the total mass of the composition, in which the composition has a solid content of 90% by mass or more with respect to the total mass of the composition.

In addition, the composition contains 35% by mass or more of the pulverulent phase (filler, pearl brightener, inorganic pigment, reflective particle), contains the organic lake in the total amount of 0.01% to 20% by mass with respect to the total mass of the composition, includes, as emulsifier, saccharide esters and ethers, fatty acid esters, oxyalkylenated alcohols, fatty alcohols, and silicone compounds, and has a solid content between 0.5% and 8% with respect to the total weight of the composition, and the hydrophilic gelling agent can be selected from a thickening filler, a polymer thickener, and an associative polymer. In addition, it is described that the composition of a specific embodiment preferably contains a chelating agent selected from aminocarboxylic acids such as EDTA tetrasodium.

PTL 4 describes a cosmetic composition in the form of a water-in-oil solid emulsion in which an aqueous phase is dispersed in a lipid phase, characterized in that the lipid phase contains at least one wax that is a solid form in the form of needle-like crystals having a melting point of 25°C to 42°C, an average length of 0.1 um to 50 um, and a shape coefficient of 2 or more, and the composition has the maximum force measured by texturometry during insertion of a probe is 0.25 newton or more.

In addition, it is described that the composition contains 50% by weight of water or an organic solvent compatible with water as aqueous phase, and contains 2% to 65% of liquid oil as lipid phase. Furthermore, it is described that the composition may also contain a particle phase, the particle phase is present in an amount of 0.01% to 40% by weight, more preferably 0.01% to 30% by weight, and particularly 0.05% to 20% by weight in the total weight of the composition, and the composition contains, as the particle phase, a pigment and/or a pearl component and/or a filler, which is commonly used particularly in cosmetic composition. In addition, it is described that the pigment means a white or colored mineral or organic particle that is insoluble in a liquid hydrophilic phase and is used to impart color and/or opacity to the composition, the filler means colorless or white, mineral or synthetic, flaky or non-flaky particles, and the pearl component means pearlescent particles that are synthetic or produced from mollusk having a shell.

PTL 5 describes a cosmetic composition containing (A) a powder composed of Nε-monooctanoyl lysine, (B) an inorganic powder, and (C) an oil agent, in which the content of the component (A) is 0.1% to 95% by weight of the whole cosmetic composition, the content of the component (B) is 4% to 99.5% by weight of the whole cosmetic composition, the content of the component (C) is 0.01% to 95% by weight of the whole cosmetic composition, the blending ratio of the component (A) and the component (B) (weight of the component (A): weight of the component (B)) is 1:99 to 60:40, and the blending ratio of the component (A) and the component (C) (weight of the component (A): weight of the component (C)) is 15:85 to 75:25.

It is described that examples of the inorganic powder include yellow iron oxide, red iron oxide, black iron oxide, fine particle iron oxide, bismuth oxychloride, carbon black and zinc oxide, and the oil agent is not particularly limited as long as it is used in a cosmetic and examples thereof include vaseline, lanolin, and ceresin.

Citation List

Patent Literature

PTL 1: JP 4684970 B
PTL 2: JP 2858020 B
PTL 3: JP 2017-190343 A
PTL 4: JP 4185018 B
PTL 5: JP 6566002 B

Summary of Invention

Technical Problem

However, titanium dioxide is potentially a carcinogenic substance and is classified into carcinogenic category 2 according to the CLP regulations in the EU, and thus it is expected that the use in a cosmetic is to be restricted eventually.

An object of the present invention is to provide a powder having excellent hiding power, such that the powder can be used in place of titanium dioxide as a white pigment in a cosmetic.

Solution to Problem

To solve the above-described problem, one aspect of the present invention is to provide a powder including plate-shaped crystal particles, in which a volume-based cumulative 50% primary particle diameter (volume D50% diameter) of the powder is 0.7 um or more and 8.0 um or less, and a coefficient of variation (CV value = standard deviation/number average primary particle diameter) of the primary particle diameter is 1.0 or less.

Advantageous Effects of Invention

According to the present invention, a powder having excellent hiding power, such that the powder can be used in place of titanium dioxide as a white pigment in a cosmetic, can be provided.

Description of Embodiments

Hereinafter, embodiments of the present invention will be described, but the present invention is not limited to the embodiments shown below. In the embodiments shown below, technically preferable limitations are made for carrying out the present invention, but the limitations are not an essential requirement of the present invention.

[First embodiment]

The white pigment for a cosmetic of this embodiment contains titanium phosphate powder. This titanium phosphate powder includes titanium phosphate crystal particles. This titanium phosphate crystal particles are plate-shaped crystal particles.

A value obtained by measuring the longest diagonal line of the plate surface of this plate-shaped crystal as the primary particle diameter by an image analysis method and calculating a volume-based cumulative 50% primary particle diameter (volume D50% diameter) is 0.7 um or more and 8.0 um or less, and a coefficient of variation (CV value = standard deviation/number average primary particle diameter) of the primary particle diameter is 1.0 or less. In addition, a value obtained by measuring the thickness of the side surface of this plate-shaped crystal and calculating the volume-based cumulative 50% thickness (volume D50% thickness) is 0.01 um or more and less than 1.00 um, and the aspect ratio (a value obtained by dividing the volume D50% diameter by the volume D50% thickness) is 5 or more.

The white pigment for a cosmetic of this embodiment is composed of titanium phosphate powder including plate-shaped crystal particles of titanium phosphate, the volume D50% diameter of the titanium phosphate powder is 0.7 um or more and 8.0 um or less, and the coefficient of variation (CV value) of the primary particle diameter of the titanium phosphate powder is 1.0 or less. Therefore, the cosmetic containing the pigment is excellent in hiding power. In addition, if the volume D50% thickness of the plate-shaped crystal particles is less than 0.01 um, the plate-shaped particles are not formed, and if the volume D50% thickness exceeds 1.00 um, the hiding power is lowered, and thus the thickness of the plate-shaped crystal particles (the volume D50% thickness) is set to 0.01 um or more and 1.00 um or less. Furthermore, since the aspect ratio is 5 or more, the slipperiness is also excellent.

This titanium phosphate powder can be obtained, for example, by the following method.

First, an aqueous solution of titanyl sulfate and an aqueous solution of phosphoric acid are mixed at a ratio in which a ratio [P]/[Ti] of a molar concentration of phosphorus [P] to a molar concentration of titanium [Ti] is to be 5 or more and 21 or less to obtain a mixed solution. Next, this obtained mixed solution is put in a sealed container, the temperature is maintained at a value within a range of 100°C or more and 160°C or less, and the reaction is carried out for a predetermined time (for example, 5 hours or more). That is, hydrothermal synthesis is performed. The pressure inside the sealed container is a pressure equal to or more than an atmospheric pressure, which is naturally determined by the pressing temperature. As a result, a slurry containing crystal particles of titanium phosphate is obtained.

Next, after cooling the obtained slurry, the solid content (crystal particles of titanium phosphate) is separated from the slurry. The obtained solid content is cleaned with a cleaning liquid consisting of water or aqueous ammonia (ammonium hydroxide) and then dried.

[Second embodiment]

The white pigment for a cosmetic of this embodiment contains boron nitride powder. The boron nitride powder includes boron nitride crystal particles. The boron nitride crystal particles are plate-shaped crystal particles.

A value obtained by measuring the longest diagonal line of the plate surface of this plate-shaped crystal as the primary particle diameter by an image analysis method and calculating a volume-based cumulative 50% primary particle diameter (volume D50% diameter) is 0.7 um or more and 8.0 um or less, and a coefficient of variation (CV value = standard deviation/number average primary particle diameter) of the primary particle diameter is 1.0 or less. In addition, a value obtained by measuring the thickness of the side surface of this plate-shaped crystal and calculating the volume-based cumulative 50% thickness (volume D50% thickness) is 0.01 um or more and less than 1.00 um, and the aspect ratio (a value obtained by dividing the volume D50% diameter by the volume D50% thickness) is 5 or more.

Similar to the cosmetic containing the white pigment for a cosmetic of the first embodiment, the cosmetic containing the white pigment for a cosmetic of this embodiment is excellent in hiding power and also excellent in slipperiness.

The white pigment for a cosmetic of this embodiment can be obtained by pulverizing a commercially available boron nitride powder.

[About cosmetic]

As a cosmetic including a composition containing a powdery white pigment (hereinafter, referred to as "cosmetic composition"), make-up cosmetics such as foundation, face powder, cheek rouge, eye color, manicure, and lipstick, and skin care cosmetics such as whitening powder and body powder can be exemplified. Since the white pigments of the first and second embodiments have a large hiding power, they are suitable as white pigments in these cosmetic compositions.

In addition, examples of cosmetic include those described in PTLs 2 to 5, and the powder according to one aspect of the present invention can be used as the fine particle powders constituting the oil-based solid cosmetic of PTL 2, as the inorganic pigment of the pulverulent phase constituting the solid make-up and/or care cosmetic composition in the form of a compact powder of PTL 3, as the pigment of the particle phase constituting the cosmetic composition in the form of a water-in-oil solid emulsion of PTL 4, and as the inorganic powder constituting the cosmetic composition of PTL 5.

In addition, the powder according to one aspect of the present invention (for example, the titanium phosphate powder of the first embodiment and the boron nitride powder of the second embodiment) can be subjected to various polymer treatments, the following treatment, or the like to improve cosmetic properties and pigment properties. Examples of these treatment methods include fluorine treatment, silicon treatment, alkylsilane treatment, alkyl titanate treatment, metal soap treatment, lauroyl lysine treatment, ester treatment, and amino acid treatment. In the amino acid treatment, proline, hydroxyproline, alanine, glycine, sarcosine, aspartic acid, and glutamic acid can be used.

A cosmetic containing a white pigment made of the powder according to one aspect of the present invention (for example, the titanium phosphate powder of the first embodiment and the boron nitride powder of the second embodiment) can contain other components as necessary within a range that the effects of the present invention are not impaired. Examples of other components include a component commonly used in a cosmetic, such as solvents, oil agents, surfactants, moisturizers, organic UV absorbers, antioxidants, thickeners, fragrances, colorants, physiologically active components, and antibacterial agents.

As the other components, one type may be used alone, or two or more types may be used in combination. The content of the other components is not particularly limited and can be appropriately set according to the purpose.

The content of the powder according to one aspect of the present invention (for example, the titanium phosphate powder of the first embodiment and the boron nitride powder of the second embodiment) contained in the cosmetic is preferably 0.1% by mass or more and 50% by mass or less with respect to the whole cosmetic. Examples

[Synthesis of titanium phosphate]

Six types (A to F) of titanium phosphate were synthesized by the following methods.

<Synthetic product A>

First, an aqueous solution of titanyl sulfate and an aqueous solution of phosphoric acid were mixed at a ratio in which a ratio [P]/[Ti] of a molar concentration of phosphorus [P] to a molar concentration of titanium [Ti] was to be 9.0 to obtain a mixed solution. Next, this obtained mixed solution was put in a 1.4 L autoclave, the temperature was maintained at 110°C, and the reaction was carried out for 5 hours.

After the reaction, the lid was opened to cool the slurry in the container to room temperature, and then the slurry was taken out from the container and filtered to separate the solid content from the slurry. This solid content was cleaned with water and then dried (left at a temperature of 105°C for 24 hours) to obtain a powder.

As a result of analyzing the obtained powder using an X-ray diffraction apparatus, it was confirmed that the particles constituting the powder were crystalline titanium phosphate having a structural formula of $Ti(HPO_4)_2 \cdot H_2O$.

When the obtained powder was observed with a scanning electron microscope, it was confirmed that the shape of the particles constituting the powder was plate-shaped shape, and that many of the particles were hexagonal plate-shaped shape. When the volume D50% diameter, CV value (Standard deviation/number average primary particle diameter), and volume D50% thickness of the crystal particles constituting the obtained powder were measured by analyzing the image of the scanning electron microscope using the image analysis software "Mac-View ver.4" manufactured by Mountech Co., Ltd., the volume D50% diameter was 0.29 um, the CV value was 0.45, and the volume D50% thickness was 0.030 um.

In addition, the aspect ratio of the crystal particles constituting the obtained powder was 10 by calculation using the measured values of the volume D50% thickness and the volume D50% diameter (0.29/0.030).

<Synthetic product B>

First, an aqueous solution of titanyl sulfate and an aqueous solution of phosphoric acid were mixed at a ratio in which a ratio [P]/[Ti] of a molar concentration of phosphorus [P] to a molar concentration of titanium [Ti] was to be 10.7 to obtain a mixed solution. Next, this obtained mixed solution was put in a 1.4 L autoclave, the temperature was maintained at 110°C, and the reaction was carried out for 5 hours.

After the reaction, the lid was opened to cool the slurry in the container to room temperature, and then the slurry was taken out from the container and filtered to separate the solid content from the slurry. This solid content was cleaned with water and then dried (left at a temperature of 105°C for 24 hours) to obtain a powder.

As a result of analyzing the obtained powder using an X-ray diffraction apparatus, it was confirmed that the particles constituting the powder were crystalline titanium phosphate having a structural formula of $Ti(HPO_4)_2 \cdot H_2O$.

When the obtained powder was observed with a scanning electron microscope, it was confirmed that the shape of the particles constituting the powder was plate-shaped shape, and that many of the particles were hexagonal plate-shaped shape. In addition, when the volume D50% diameter, CV value (Standard deviation/number average primary particle diameter), and volume D50% thickness of the crystal particles constituting the obtained powder were measured by the same manner as the synthetic product A, the volume D50% diameter was 0.53 um, the CV value was 0.34, and the volume D50% thickness was 0.065 um.

Further, the aspect ratio of the crystal particles constituting the obtained powder was 8 by calculation using the measured values of the volume D50% thickness and the volume D50% diameter (0.53/0.065).

<Synthetic product C>

First, an aqueous solution of titanyl sulfate and an aqueous solution of phosphoric acid were mixed at a ratio in which a ratio [P]/[Ti] of a molar concentration of phosphorus [P] to a molar concentration of titanium [Ti] was to be 10.4 to obtain a mixed solution. Next, this obtained mixed solution was put in a 1.4 L autoclave, the temperature was maintained at 110°C, and the reaction was carried out for 5 hours.

After the reaction, the lid was opened to cool the slurry in the container to room temperature, and then the slurry was taken out from the container and filtered to separate the solid content from the slurry. This solid content was cleaned with water and then dried (left at a temperature of 105°C for 24 hours) to obtain a powder.

As a result of analyzing the obtained powder using an X-ray diffraction apparatus, it was confirmed that the particles constituting the powder were crystalline titanium phosphate having a structural formula of $Ti(HPO_4)_2 \cdot H_2O$.

When the obtained powder was observed with a scanning electron microscope, it was confirmed that the shape of the particles constituting the powder was plate shape, and that many of the particles were hexagonal plate shape. In addition, when the volume D50% diameter, CV value (Standard deviation/number average primary particle diameter), and volume D50% thickness of the crystal particles constituting the obtained powder were measured by the same manner as the synthetic product A, the volume D50% diameter was 0.74 um, the CV value was 0.42, and the volume D50% thickness was 0.090 um.

Further, the aspect ratio of the crystal particles constituting the obtained powder was 8 by calculation using the measured values of the volume D50% thickness and the volume D50% diameter (0.74/0.090).

<Synthetic product D>

First, an aqueous solution of titanyl sulfate and an aqueous solution of phosphoric acid were mixed at a ratio in which a ratio [P]/[Ti] of a molar concentration of phosphorus [P] to a molar concentration of titanium [Ti] was to be 10.2 to obtain a mixed solution. Next, this obtained mixed solution was put in a 200 L autoclave, the temperature was maintained at 110°C, and the reaction was carried out for 5 hours.

After the reaction, the lid was opened to cool the slurry in the container to room temperature, and then the slurry was taken out from the container and filtered to separate the solid content from the slurry. This solid content was cleaned with 29% aqueous ammonia (an aqueous solution of an ammonium salt) and then dried (left at a temperature of 105°C for 24 hours) to obtain a powder.

As a result of analyzing the obtained powder using an X-ray diffraction apparatus, it was confirmed that the particles constituting the powder were crystalline titanium phosphate having a structural formula of $Ti(HPO_4)_2 \cdot H_2O$.

When the obtained powder was observed with a scanning electron microscope, it was confirmed that the shape of the particles constituting the powder was plate shape, and that many of the particles were hexagonal plate shape. In addition, when the volume D50% diameter, CV value (Standard deviation/number average primary particle diameter), and volume D50% thickness of the crystal particles constituting the obtained powder were measured by the same manner as the synthetic product A, the volume D50% diameter was 1.11 um, the CV value was 0.33, and the volume D50% thickness was 0.143 um.

Further, the aspect ratio of the crystal particles constituting the obtained powder was 8 by calculation using the measured values of the volume D50% thickness and the volume D50% diameter (1.11/0.143).

<Synthetic product E>

First, an aqueous solution of titanyl sulfate and an aqueous solution of phosphoric acid were mixed at a ratio in which a ratio [P]/[Ti] of a molar concentration of phosphorus [P] to a molar concentration of titanium [Ti] was to be 6.9 to obtain a mixed solution. Next, this obtained mixed solution was put in a 1.4 L autoclave, the temperature was maintained at 120°C, and the reaction was carried out for 5 hours.

After the reaction, the lid was opened to cool the slurry in the container to room temperature, and then the slurry was taken out from the container and filtered to separate the solid content from the slurry. This solid content was cleaned with water and then dried (left at a temperature of 105°C for 24 hours) to obtain a powder.

As a result of analyzing the obtained powder using an X-ray diffraction apparatus, it was confirmed that the particles constituting the powder were crystalline titanium phosphate having a structural formula of $Ti(HPO_4)_2 \cdot H_2O$.

When the obtained powder was observed with a scanning electron microscope, it was confirmed that the shape of the particles constituting the powder was plate shape, and that many of the particles were hexagonal plate shape. In addition, when the volume D50% diameter, CV value (Standard deviation/number average primary particle diameter), and volume D50% thickness of the crystal particles constituting the obtained powder were measured by the same manner as the synthetic product A, the volume D50% diameter was 2.07 um, the CV value was 0.37, and the volume D50% thickness was 0.302 um.

Further, the aspect ratio of the crystal particles constituting the obtained powder was 7 by calculation using the measured values of the volume D50% thickness and the volume D50% diameter (2.07/0.302).

<Synthetic product F>

First, an aqueous solution of titanyl sulfate and an aqueous solution of phosphoric acid were mixed at a ratio in which a ratio [P]/[Ti] of a molar concentration of phosphorus [P] to a molar concentration of titanium [Ti] was to be 10.8 to obtain a mixed solution. Next, this obtained mixed solution was put in a 200 L autoclave, the temperature was maintained at 130°C, and the reaction was carried out for 5 hours.

After the reaction, the lid was opened to cool the slurry in the container to room temperature, and then the slurry was taken out from the container and filtered to separate the solid content from the slurry. This solid content was cleaned with water and then dried (left at a temperature of 105°C for 24 hours) to obtain a powder.

As a result of analyzing the obtained powder using an X-ray diffraction apparatus, it was confirmed that the particles constituting the powder were crystalline titanium phosphate having a structural formula of $Ti(HPO_4)_2 \cdot H_2O$.

When the obtained powder was observed with a scanning electron microscope, it was confirmed that the shape of the particles constituting the powder was plate shape, and that many of the particles were hexagonal plate shape. In addition, when the volume D50% diameter, CV value (Standard deviation/number average primary particle diameter), and volume D50% thickness of the crystal particles constituting the obtained powder were measured by the same manner as the synthetic product A, the volume D50% diameter was 7.44 um, the CV value was 0.36, and the volume D50% thickness was 0.856 um.

Further, the aspect ratio of the crystal particles constituting the obtained powder was 9 by calculation using the measured values of the volume D50% thickness and the volume D50% diameter (7.44/0.856).

[Preparation of Powder]

The synthesized titanium phosphate powders of A to F were mixed at the ratios shown in Table 1 to obtain titanium phosphate powders of No. 1 to No. 9. The titanium phosphate powders of No. 1 to No. 3, and No. 7 are the same as the titanium phosphate powders of the synthetic products of A to D, respectively. Since the titanium phosphate powders of No. 4 to No. 6, No. 8, and No. 9 are mixed products, the volume D50% diameter, the CV value, and the volume D50% thickness were measured by the above-described methods, and the aspect ratio was calculated.

In addition, commercially available boron nitride powder (No. 10) having a volume D50% diameter of 9.94 um was prepared, and pulverized with a pot mill, and boron nitride powders of No. 11 to No. 14 having the constitution shown in Table 2 were obtained. For these boron nitride powders, the volume D50% diameter, the CV value, and the volume D50% thickness were also measured by the above-described methods, and the aspect ratio was calculated.

Furthermore, a commercially available titanium dioxide powder (No. 15) was prepared.

In addition, the titanium phosphate powder No. 16 was synthesized by the following method.

First, an aqueous solution of titanyl sulfate and an aqueous solution of phosphoric acid were mixed at a ratio in which a ratio [P]/[Ti] of a molar concentration of phosphorus [P] to a molar concentration of titanium [Ti] was to be 18.1 to obtain a mixed solution. Next, this obtained mixed solution was put in a 200 L autoclave, the temperature was maintained at 130°C, and the reaction was carried out for 10 hours.

After the reaction, the lid was opened to cool the slurry in the container to room temperature, and then the slurry was taken out from the container and filtered to separate the solid content from the slurry. This solid content was cleaned with water and then dried (left at a temperature of 105°C for 24 hours) to obtain a powder.

As a result of analyzing the obtained powder using an X-ray diffraction apparatus, it was confirmed that the particles constituting the powder were crystalline titanium phosphate having a structural formula of $Ti(HPO_4)_2 \cdot H_2O$.

When the obtained powder was observed with a scanning electron microscope, it was confirmed that the shape of the particles constituting the powder was plate shape, and that many of the particles were hexagonal plate shape. In addition, when the volume D50% diameter, CV value (Standard deviation/number average primary particle diameter), and volume D50% thickness of the crystal particles constituting the obtained powder were measured by the same manner as the synthetic product A, the volume D50% diameter was 8.87 um, the CV value was 0.38, and the volume D50% thickness was 0.930 um.

Further, the aspect ratio of the crystal particles constituting the obtained powder was 10 by calculation using the measured values of the volume D50% thickness and the volume D50% diameter (8.87/0.930).

Table 2 shows the volume D50% diameter, the CV value, the volume D50% thickness, and the aspect ratio of each powder.

[Measurement of Hiding Power]

The hiding power of each of the powders of No. 1 to No. 16 was measured by the following method.

First, 1.4 g of ethanol and 5.6 g of a 10% collodion solution were added to 3 g of each powder and mixed to obtain a slurry. Next, the obtained slurry was applied and dried on a black-and-white hiding power test chart with a film thickness of 100 um in accordance with JIS K5600-4 (General testing methods for paints - testing method related to visual characteristics of coating films) to obtain test sample.

Next, using a spectral colorimeter ("CM-512m3A" manufactured by Konica Minolta, Inc.), light incident on the obtained test sample at an angle of 45° was received just above (0°) to perform color measurement on a white background and a black background, and the color difference ($\Delta E$) in the L*a*b* color system was calculated by Equation (1) . ($L^*_1$, $a^*_1$, $b^*_1$) is a color measurement value on a black background, and ($L^*_2$, $a^*_2$, $b^*_2$) is a color measurement value on a white background. The hiding power was calculated from Equation (2) using $\Delta E$. The results are also shown in Table 2.

$$\Delta E = \sqrt{\{(L^*_2 - L^*_1)^2 + (a^*_2 - a^*_1)^2 + (b^*_2 - b^*_1)^2\}} \quad \ldots \quad (1)$$

$$\text{Hiding power} = 100/\Delta E \quad \ldots \quad (2)$$

[Table 1]

|  | A | B | C | D | E | F |
|---|---|---|---|---|---|---|
| Volume D50% diameter [$\mu$m] | 0.29 | 0.53 | 0.74 | 1.11 | 2.07 | 7.44 |
| Volume D50% thickness [$\mu$m] | 0.030 | 0.065 | 0.090 | 0.143 | 0.302 | 0.856 |
| No. 1 | 100 | 0 | 0 | 0 | 0 | 0 |
| No. 2 | 0 | 100 | 0 | 0 | 0 | 0 |
| No. 3 | 0 | 0 | 100 | 0 | 0 | 0 |
| No. 4 | 0 | 10 | 5 | 15 | 70 | 0 |
| No. 5 | 5 | 18 | 0 | 77 | 0 | 0 |
| No. 6 | 4 | 0 | 0 | 35 | 0 | 61 |
| No. 7 | 0 | 0 | 0 | 100 | 0 | 0 |
| No. 8 | 0 | 0 | 0 | 40 | 60 | 0 |
| No. 9 | 0 | 0 | 0 | 35 | 50 | 15 |

[Table 2]

| No. | Constitution | | | | | Ability |
|---|---|---|---|---|---|---|
|  | Material | Volume D50% diameter [$\mu$m] | CV value | Volume D50% thickness [$\mu$m] | Aspect ratio | Hiding power |
| 1 | Titanium phosphate | <u>0.29</u> | 0.45 | 0.030 | 10 | 15 |
| 2 | Titanium phosphate | <u>0.53</u> | 0.34 | 0.065 | 8 | 14 |
| 3 | Titanium phosphate | 0.74 | 0.42 | 0.090 | 8 | 20 |
| 4 | Titanium phosphate | 1.89 | 0.82 | 0.263 | 7 | 20 |
| 5 | Titanium phosphate | 0.97 | 0.44 | 0.123 | 8 | 29 |
| 6 | Titanium phosphate | 6.23 | <u>1.05</u> | 0.768 | 8 | 7 |

(continued)

| No. | Constitution | | | | | Ability |
| | Material | Volume D50% diameter [μm] | CV value | Volume D50% thickness [μm] | Aspect ratio | Hiding power |
|---|---|---|---|---|---|---|
| 7 | Titanium phosphate | 1.11 | 0.33 | 0.143 | 8 | 36 |
| 8 | Titanium phosphate | 1.97 | 0.48 | 0.273 | 7 | 22 |
| 9 | Titanium phosphate | 7.01 | 0.67 | 0.950 | 7 | 20 |
| 10 | Boron nitride | 9.94 | 0.43 | 2.088 | 5 | 18 |
| 11 | Boron nitride | 0.35 | 0.49 | 0.121 | 3 | 9 |
| 12 | Boron nitride | 1.84 | 0.73 | 0.366 | 5 | 29 |
| 13 | Boron nitride | 4.24 | 0.87 | 0.395 | 11 | 25 |
| 14 | Boron nitride | 1.22 | 0.59 | 0.173 | 7 | 23 |
| 15 | Titanium dioxide | 0.38 | 0.29 | Not plate-shaped crystal | | 34 |
| 16 | Titanium phosphate | 8.87 | 0.38 | 0.930 | 10 | 12 |

From this result, the following can be understood.

The titanium phosphate powder and the boron nitride powder of No. 3 to No. 5, No. 7 to No. 9, and No. 12 to No. 14, which include plate-shaped crystal particles and in which a volume D50% diameter is 0.7 um or more and 8.0 um or less, a coefficient of variation (CV value) of a primary particle diameter is 1.0 or less, and a volume D50% thickness is 0.01 um or more and less than 1.00 um, have a high hiding power of 20 or more and 36 or less. In particular, the hiding power of the titanium phosphate powder of No. 7 was higher than that of the titanium dioxide powder of No. 15. On the other hand, the hiding power of the titanium phosphate powders of No. 1 and No. 2 and the boron nitride powder of No. 11 in which a volume D50% diameter is less than 0.7 um, the titanium phosphate powder of No. 6 in which a CV value is more than 1.0, the titanium phosphate powder of No. 16 in which a volume D50% diameter is more than 8.0 um, and the boron nitride powder of No. 10 in which a volume D50% diameter is more than 8.0 um, are 15, 14, 9, 7, 12, and 18, respectively, which are low.

That is, the titanium phosphate powders and the boron nitride powders of No. 3 to No. 5, No. 7 to No. 9, and No. 12 to No. 14 are powders which are excellent in a hiding power such that they can be used in a cosmetic as white pigment in place of titanium dioxide. In addition, when the aspect ratio is 5 or more, an effect that the slipperiness is excellent is obtained.

Furthermore, the titanium phosphate powder and the boron nitride powder of No. 5, No. 7, and No. 12 have a particularly high hiding power of 29 or more. That is, a powder which includes plate-shaped crystal particles and in which a volume-based cumulative 50% primary particle diameter (volume D50% diameter) is 0.97 um or more and 1.84 um or less, is preferable due to particularly high hiding power. In addition, a titanium phosphate powder which includes plate-shaped crystal particles and in which a volume-based cumulative 50% thickness (volume D50% thickness) of the plate-shaped crystal particles is 0.142 um or more and 0.143 um or less, is preferable due to particularly high hiding power.

[Preparation of Cosmetic Composition]

When a cosmetic composition containing the titanium phosphate powders and the boron nitride powders of No. 3 to No. 5, No. 7 to No. 9, and No. 12 to No. 14 as white pigments was prepared, the effects of covering the skin color and adjusting the skin color were improved due to high hiding power.

[Measurement of Whiteness, Refractive Index, Oil Absorption Amount, and Specific Surface Area]

The whiteness, refractive index, oil absorption amount, and specific surface area of each of the powders No. 1 to No. 16 were measured by the following methods.

The whiteness was measured using an ultraviolet-visible spectrophotometer "UV-2450" manufactured by Shimadzu Corporation under the conditions of illumination D65 and a field of view of 2°. That is, the whiteness of each powder was measured by a method in accordance with JIS Z 8715.

For the refractive index, first, each of the obtained powders and polymethyl methacrylate (film substrate: a transparent resin that serves as a film base) are put into N-methylpyrrolidone (a solvent in which the film substrate can be dissolved) and mixed, and then the powder was dispersed to obtain a liquid in which polymethyl methacrylate was dissolved. A plurality of these liquids were obtained by changing the content of the powder. Using these liquids, a coating film having a thickness of 600 um was formed on a PET film and dried at 80°C to form a film consisting of only powder and resin. After cooling, the film was peeled off from the PET film.

The refractive indices of the plurality of films thus obtained were measured using a helium neon laser beam having a wavelength of 632.8 nm as a light source using a refractive meter "Prism Coupler Model 2010/M" manufactured by METRICON Corporation. The measured values of the refractive indices of the plurality of films were plotted on a graph in which the horizontal axis was the content of powder (% by volume) and the vertical axis was the refractive index, and each plot was approximated by a straight line. The value of the refractive index at the point where this straight line was extrapolated to the point where the content of the powder became 100% was defined as the refractive index of the powder.

The oil absorption amount was measured per 100 g by a method in accordance with JIS K 5101-13. The specific surface area was measured by the BET flow method using a fully automatic specific surface area measuring device "Macsorb (registered trademark) HM-1210" manufactured by Mountech Co., Ltd. In addition, using these measured values, the ratio (oil absorption amount/specific surface area) of the oil absorption amount (ml/100 g) to the specific surface area ($m^2/g$) of the crystal particles in each powder was calculated.

The above results are shown in Table 3. SA in Table 3 indicates the specific surface area.

[Table 3]

| No. | Material | Physical property | | | | |
|---|---|---|---|---|---|---|
| | | Whiteness | Refractive index | Oil absorption amount (ml/100 g) | SA ($m^2/g$) | Oil Absorption amount/SA |
| 1 | Titanium phosphate | 100.26 | 1.79 | 140 | 31.3 | 4.5 |
| 2 | Titanium phosphate | 96.80 | 1.76 | 90 | 15.7 | 5.7 |
| 3 | Titanium phosphate | 99.56 | 1.78 | 80 | 11.6 | 6.9 |
| 4 | Titanium phosphate | 99.35 | 1.79 | 58 | 6.2 | 9.4 |
| 5 | Titanium phosphate | 97.80 | 1.79 | 88 | 7.7 | 11.4 |
| 6 | Titanium phosphate | 96.80 | 1.72 | 55 | 4.0 | 13.8 |
| 7 | Titanium phosphate | 99.00 | 1.74 | 90 | 6.9 | 13.0 |
| 8 | Titanium phosphate | 99.13 | 1.78 | 63 | 5.2 | 12.1 |
| 9 | Titanium phosphate | 98.74 | 1.75 | 60 | 4.6 | 13.0 |
| 10 | Boron nitride | 96.54 | <u>1.81</u> | 116 | 2.7 | 43.0 |
| 11 | Boron nitride | 92.13 | <u>1.82</u> | 70 | 20.4 | 3.4 |
| 12 | Boron nitride | <u>91.24</u> | <u>1.81</u> | 87 | 13.0 | 6.7 |

(continued)

| No. | Material | Physical property | | | | |
|---|---|---|---|---|---|---|
| | | Whiteness | Refractive index | Oil absorption amount (ml/ 100 g) | SA (m$^2$/g) | Oil Absorption amount/SA |
| 13 | Boron nitride | 98.47 | <u>1.82</u> | 100 | 12.7 | 7.9 |
| 14 | Boron nitride | 98.42 | <u>1.83</u> | 155 | 37.9 | 4.1 |
| 15 | Titanium dioxide | <u>90.68</u> | <u>2.10</u> | 35 | 10.0 | 3.5 |
| 16 | Titanium phosphate | 92.99 | 1.71 | 76 | 1.7 | 44.7 |

From this result, the following can be understood.

Each of the powders of No. 1 to No. 9, and No. 16 has a high whiteness of 92.91 or more measured in accordance with JIS Z 8715, and thus can exhibit a high function as a base pigment. In addition, each of the powders of No. 1 to No. 9, and No. 16 has a refractive index of 1.67 or more and less than 1.80, which is moderately higher than the refractive index of human skin (1.5), and thus it is possible to obtain a natural finish that does not cause whitening with an appropriate covering power by using the cosmetic composition in which each of the powders is blended. In addition, each of the powders of No. 1 to No. 9, and No. 16 has a ratio (oil absorption amount/SA) of an oil absorption amount (ml/100 g) to the specific surface area (m$^2$/g) of the crystal particles of 4.0 or more, and thus the cosmetic composition in which each of the powders is blended, has less stickiness of the coating film and has a long-lasting cosmetic appearance.

**Claims**

1.  A powder comprising:

    plate-shaped crystal particles,
    wherein a volume-based cumulative 50% primary particle diameter (volume D50% diameter) of the powder is 0.7 um or more and 8.0 um or less, and
    a coefficient of variation (CV value) of the primary particle diameter is 1.0 or less.

2.  The powder according to claim 1, wherein a volume-based cumulative 50% thickness (volume D50% thickness) of the plate-shaped crystal particles is 0.01 um or more and less than 1.00 um, and
    an aspect ratio is 5 or more, the aspect ratio being a value obtained by dividing the volume-based cumulative 50% primary particle diameter of the plate-shaped crystal particles by the volume-based cumulative 50% thickness.

3.  The powder according to claim 1 or 2, wherein the plate-shaped crystal particles comprising titanium phosphate.

4.  The powder according to any one of claims 1 to 3, wherein the plate-shaped crystal particles are a hexagonal plate-shaped crystal particles.

5.  A white pigment for a cosmetic comprising the powder according to any one of claims 1 to 4.

6.  A cosmetic comprising a composition containing the white pigment for a cosmetic according to claim 5.

## INTERNATIONAL SEARCH REPORT

| International application No. |
| --- |
| **PCT/JP2021/033013** |

### A. CLASSIFICATION OF SUBJECT MATTER

*A61K 8/29*(2006.01)i; *A61K 8/02*(2006.01)i; *A61Q 1/12*(2006.01)i; *C01B 25/37*(2006.01)i
FI:    A61K8/29; A61K8/02; A61Q1/12; C01B25/37

According to International Patent Classification (IPC) or to both national classification and IPC

### B. FIELDS SEARCHED

Minimum documentation searched (classification system followed by classification symbols)

A61K8/00-8/99; A61Q1/00-90/00; C01B25/37; C01B21/064

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X | WO 2018/180797 A1 (FUJIMI INC.) 04 October 2018 (2018-10-04)<br>claims 1-3, 11, paragraphs [0010], [0015], table 1 | 1-6 |
| X | WO 2019/189665 A1 (FUJIMI INC.) 03 October 2019 (2019-10-03)<br>claims 1-8, paragraphs [0024], [0025], [0044]-[0049], table 1 | 1-6 |
| X | WO 2020/059191 A1 (FUJIMI INCO.) 26 March 2020 (2020-03-26)<br>claims 1-5, paragraphs [0020]-[0022], [0033]-[0038], table 1 | 1-6 |
| X | JP 2020-75845 A (TOKUYAMA CORP.) 21 May 2020 (2020-05-21)<br>claim 3, paragraphs [0017], [0054]-[0058], [0066] | 1-2, 4-6 |
| X | JP 2012-176910 A (MIZUSHIMA FERROALLOY CO., LTD.) 13 September 2012 (2012-09-13)<br>claims 1, 7-9, paragraphs [0004], [0024], table 1 | 1-2, 4-6 |
| X | WO 2019/172440 A1 (DENKA CO., LTD.) 12 September 2019 (2019-09-12)<br>claims 1, 5-6, 10, paragraphs [0002], [0042], [0043], [0070] | 1, 4-6 |

☑ Further documents are listed in the continuation of Box C.      ☑ See patent family annex.

| | |
| --- | --- |
| *    Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| **08 November 2021** | **22 November 2021** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2021/033013**

**C.    DOCUMENTS CONSIDERED TO BE RELEVANT**

| Category* | Citation of document, with indication, where appropriate, of the relevant passages | Relevant to claim No. |
|---|---|---|
| A | JP 10-167929 A (CATALYSTS & CHEMICALS INDUSTRIES CO., LTD.) 23 June 1998 (1998-06-23) | 1-6 |
| A | JP 2000-229809 A (NIPPON SHIKIZAI INC.) 22 August 2000 (2000-08-22) | 1-6 |

Form PCT/ISA/210 (second sheet) (January 2015)

**INTERNATIONAL SEARCH REPORT**
Information on patent family members

International application No.

**PCT/JP2021/033013**

| Patent document cited in search report | | | Publication date (day/month/year) | Patent family member(s) | Publication date (day/month/year) |
|---|---|---|---|---|---|
| WO | 2018/180797 | A1 | 04 October 2018 | US 2020/0377369 A1<br>claims 1-3, 11, paragraphs [0026], [0033]-[0035], page 3, table 1<br>EP 3604218 A1<br>KR 10-2019-0113990 A<br>CN 110461765 A | |
| WO | 2019/189665 | A1 | 03 October 2019 | US 2021/0145712 A1<br>claims 1-3, 5-9, paragraphs [0051]-[0063], [0088]-[0098], table 1<br>EP 3777819 A1 | |
| WO | 2020/059191 | A1 | 26 March 2020 | (Family: none) | |
| JP | 2020-75845 | A | 21 May 2020 | (Family: none) | |
| JP | 2012-176910 | A | 13 September 2012 | (Family: none) | |
| WO | 2019/172440 | A1 | 12 September 2019 | (Family: none) | |
| JP | 10-167929 | A | 23 June 1998 | (Family: none) | |
| JP | 2000-229809 | A | 22 August 2000 | (Family: none) | |

Form PCT/ISA/210 (patent family annex) (January 2015)

**EP 4 215 176 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4684970 B
- JP 2858020 B
- JP 2017190343 A
- JP 4185018 B
- JP 6566002 B